# EUROPEAN PATENT APPLICATION

(11) **EP 3 192 553 A1**
(43) Date of publication of application: **19.07.2017**
(21) Application number: 16849926.7
(22) Date of filing: 15.06.2016
(51) Int. Cl.: A61M 25/10, A61M 29/00, A61B 1/32

(54) **BRAIN RETRACTION DEVICE AND SURGICAL RETRACTION DEVICE**

(30) Priority: 16.11.2015 CN 201510783116; 19.05.2016 CN 201610334312
(71) Applicant: Shineyard Medical Device Co., Ltd., Shenzhen, Guangdong 518101 (CN)
(72) Inventor: WANG, Feng, Shenzhen Guangdong 518101 (CN); WANG, Yuhui, Shenzhen Guangdong 518101 (CN)
(74) Representative: Roman, Alexis
(86) International application number: PCT/CN2016/085862
(87) International publication number: WO 2017/084328

(57) **Abstract**

The present invention can be applied in the technical field of medical instruments in neurosurgery, and provides a brain retractor device comprising: a flexible retracting component which comprises an expandable sacculus, a supporting tube, a connecting base, a lock mechanism lockable and matching with the connecting base, and a pressurizing device connector configured for expanding or retracting the expandable sacculus. A brain depressor is sheathed on a distal end of the flexible retractor component and enters an avulsed passageway avulsed by the flexible retractor component so as to make the avulsed passageway remain an avulsed status. In the brain retractor device, the lock mechanism is pushed into the connecting base, and the expandable sacculus is stretched and becomes slim, such that the length and the position of the expandable sacculus that enter the diseased region are controlled. Moreover, by injecting liquid into the pressurizing device connector, the expandable sacculus is expanded; and when liquid is discharged, the expandable sacculus is retracted. By quantitatively controlling the amount of the liquid injected into the pressurizing device connector, the expanded diameter of the expandable sacculus is accurately controlled, and thus the size and the position of the avulsed passageway is accurately controlled, such that injury to brain tissue during the avulsing process can be reduced, the amount of bleeding in the surgery can be reduced, and cranial nerves can be protected to the maximum extent.

## Description

### THCHNICAL FIELD

The present application belongs to the technical field of medical instruments in neurosurgery, and more particularly relates to a brain retractor device and a surgical retractor device.

### BACKGROUND

Conventional methods for avulsing brain tissue and establishing a surgical passageway mainly rely on a brain depressor (e.g., a stainless steel plate). By virtue of manually operating the brain depressor and according to an anatomical structure of a brain, brain tissue can be avulsed. Avulsion of brain tissue relies mainly on professional skills and experience of a surgery operator. Moreover, in the process of avulsing the brain tissue, blood vessels of brain and nerves are prone to be injured, and brain retraction injuries can be seen everywhere. What's worse, after the brain tissue is avulsed, due to creep characteristic of the brain tissue, the expanded passageway is prone to be retracted and diminished, and thus the surgery operation is affected. Using the brain depressor to directly avulse the brain requires a much bigger bone window so as to perform this surgery operation, the trauma is large, an infection is prone to occur, recovery time for a patient is long, and the patient suffers from a lot of pain and bears heavier economical burden.

As for a surgery requiring avulsing brain parenchyma, the brain depressor is useless, and it is needed to use a coagulation scalpel to perform a cutting operation. However, the coagulation scalpel is prone to make unrecoverable injuries to the nerves and the blood vessels in the brain tissue, which may cause the brain to lose some functions, and even cause death of the patient.

### SUMMARY OF THE INVENTION

A purpose of the present invention is providing a brain retractor device, which aims at solving the problem of how to reduce damages to nerves and blood vessels in brain tissue caused by a brain tissue retraction surgery in the prior art.

The invention is realized as follows: a brain retractor device comprising:
a flexible retractor component which comprises an expandable sacculus that is made of elastic material and can be retracted so as to enter a diseased region and form an avulsed passageway for the diseased region; a supporting tube communicated with the expandable sacculus; a connecting base, wherein the connecting base and the expandable sacculus are located at two ends of the supporting tube respectively; a lock mechanism arranged coaxially with the supporting tube and lockable with and matching with the connecting base; a pressurizing device connector configured for expanding or retracting the expandable sacculus through the connecting base; and a brain depressor, which is sheathed on a distal end of the flexible retractor component and enters the avulsed passageway opened by the flexible retractor component so as to make the avulsed passageway remain an avulsed status.

The present invention further provides a surgical retractor device comprising:
a flexible retractor component which comprises an expandable sacculus that is made of elastic material and can be retracted so as to enter a diseased region and form an avulsed passageway for the diseased region; a supporting tube communicated with the expandable sacculus; a connecting base, wherein the connecting base and the expandable sacculus are arranged at two ends of the supporting tube; a lock mechanism arranged coaxially with the supporting tube, and lockable and matching with the connecting base; and a pressurizing device connector configured for expanding or retracting the expandable sacculus through the connecting base.

Further, the expandable sacculus is a compliant sacculus or non-compliant sacculus.

Furthermore, the expandable sacculus is cylindrical, dumbbell-shaped, peanut-shaped, or cone-shaped.

Furthermore, a proximal end of the connecting base is provided with a bayonet that matches with the lock mechanism, the lock mechanism comprises a push rod that runs through the connecting base and sequentially stretches into the supporting tube and the expandable sacculus, a pushing member connected with the push rod, and a fixture block protruded from an outer side of a front end of the pushing member so as to match with the bayonet.

Furthermore, the supporting tube and the push rod are arranged coaxially.

Furthermore, one end of the expandable sacculus is fixed to the push rod, and another end of the expandable sacculus is fixed to the supporting tube.

Furthermore, a distal end of the expandable sacculus near the flexible retractor component is fixed to the push rod through a first fastening piece, a proximal end of the expandable sacculus near the flexible retractor component is fixed to the supporting tube through a second fastening piece, and the first fastening piece and the second fastening piece are respectively sheathed on the distal end and the proximal end of the expandable sacculus.

Furthermore, a part of the distal end of the expandable sacculus near the flexible retractor component is bent toward the inside of the expandable sacculus, and the first fastening piece is sheathed on a bent part of the expandable sacculus that is inside the expandable sacculus; the second fastening piece is sheathed on an outer side of the part of the proximal end of the expandable sacculus near the flexible retractor component.

Furthermore, when the pushing member is pushed and drives the push rod to move towards the distal end of the flexible retractor component, the expandable sacculus is expanded in a length direction; when the pushing member is pushed and drives the push rod to move towards the distal end of the flexible retractor component until the fixture block is embedded in the bayonet, and when the expandable sacculus is expanded by the pressurizing device, the expandable sacculus is only expanded radially.

Furthermore, an inner part of the push rod near a distal end of the flexible retractor component is filled with inner core.

Furthermore, the connecting base comprises a sealed base body and a nut cap, both an interior of the sealed base body and an exterior of the nut cap are provided with threads, and the thread of the sealed base body and the thread of the nut cap match with each other; the nut cap is threadedly connected inside the sealed base body, the push rod stretches into the cap and the sealed base body sequentially, and a room formed by a part of the cap near the distal end of the flexible retractor component, the interior of the sealed base body, and an exterior of the push rod is provided therein with a sealing ring.

The present invention has the following technical effects over the prior art: in the brain retractor device and the surgical retractor device, the lock mechanism is pushed into the connecting base to make the expandable sacculus be stretched and becomes slim, such that the length of the expandable sacculus that enters the diseased region can be controlled. Moreover, by injecting liquid into the pressurizing device connector, the expandable sacculus is expanded, and when liquid is discharged, the expandable sacculus is retracted. By quantitatively controlling the amount of the liquid injected into the pressurizing device connector, the expanded diameter of the expandable sacculus is accurately controlled, and thus the size and the position of the avulsed passageway is accurately controlled, injury to brain tissue in the avulsing process can be reduced, the amount of bleeding in the surgery can be reduced, and cranial nerves can be protected to the maximum extent.

### BRIEF DESCRIPTION OF THE DRAWINGS

In order to explain the embodiments of the present invention more clearly, a brief introduction regarding the accompanying drawings that need to be used for describing the embodiments of the present invention or the prior art is given below; it is obvious that the accompanying drawings described as follows are only some embodiments of the present invention, for those skilled in the art, other drawings can also be obtained according to the current drawings on the premise of paying no creative labor.
FIG.1 illustrates a disassembled view of a brain retractor device provided by an embodiment of the present invention;
FIG. 2 illustrates a structural view of a flexible retractor component shown in FIG. 1;
Figures (a)-(c) in FIG. 3 illustrate a structural view of a brain depressor shown in FIG. 1;
Figures (a)-(d) in FIG. 4 illustrate a structural view of expandable sacculus shown in FIG. 2;
Figures (a)-(j) in FIG. 5 illustrate a sectional view of a supporting tube shown in FIG. 2;
Figures (a)-(h) in FIG. 6 illustrate a working state view of the brain retractor device in a first scheme of the embodiment of the present invention;
Figures (a)-(g) in FIG. 7 illustrate a working state view of the brain retractor device in a second scheme of the embodiment of the present invention;
Figures (a)-(e) in FIG. 8 illustrate a working state view of the brain retractor device in a third scheme of the embodiment of the present invention;

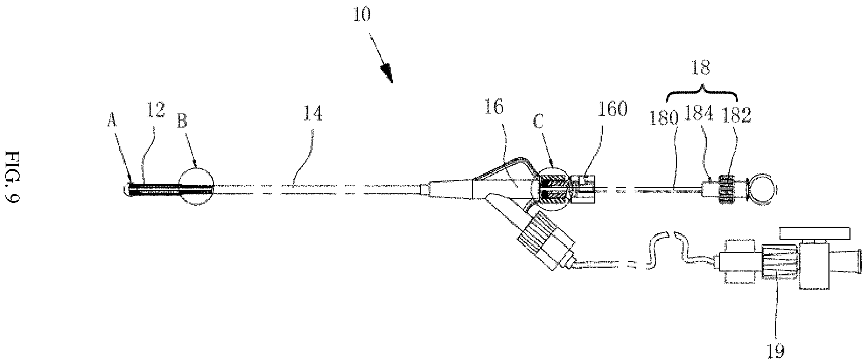

FIG. 9 illustrates a structural schematic view of a flexible retractor component of a brain retractor device provided by another embodiment of the present invention.

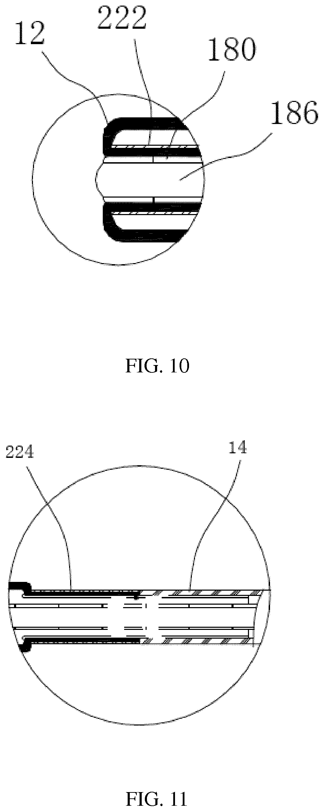

FIG. 10 illustrates an enlarged view of the part A shown in FIG. 9;
FIG. 11 illustrates an enlarged view of the part B shown in FIG. 9;
FIG. 10 illustrates an enlarged view of the part C shown in FIG. 9.

| | | | |
|---|---|---|---|
| 10 | Flexible retractor component | 180 | Push rod |
| 12 | Expandable Sacculus | 182 | Pushing Member |
| 14 | Supporting Tube | 184 | Fixture Block |
| 16 | Connecting Base | 186 | Inner Core |
| 160 | Bayonet | 19 | Pressurizing Device Connector |
| 162 | The First Cavity Opening | 20 | Brain Depressor |
| 164 | The Second Cavity Opening | 222 | The First Fastening Piece |
| 166 | The Third Cavity Opening | 224 | The Second Fastening Piece |
| 167 | Sealed Base Body | 169 | Seal Ring |
| 168 | Nut Cap | | |

### DETAILED DESCRIPTION OF THE PREFERRED EMBODIMENT

In order to make the purposes, technical solutions, and advantages of the present application be clearer and more understandable, the present invention will be further described in detail hereinafter with reference to the accompanying drawings and embodiments. It should be understood that the embodiments described herein are only intended to illustrate but not to limit the present application.

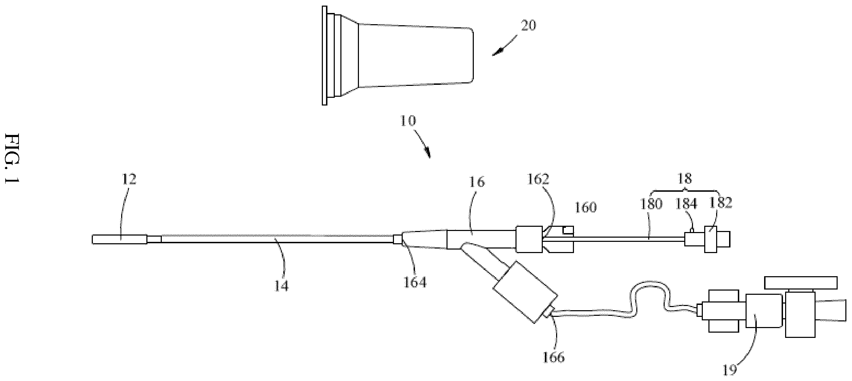

Please refer to FIGS. 1-8, a brain retractor device provided by one embodiment of the present invention comprises:
a flexible retractor component 10 which comprises an expandable sacculus 12 that is made of elastic material and can be retracted so as to enter a diseased region and form an avulsed passageway at the diseased region; a supporting tube 14 communicated with the expandable sacculus 12; a connecting base 16, wherein the connecting base 16 and the expandable sacculus 12 are located at two sides of the supporting tube 14 respectively; a lock mechanism 18 arranged coaxially with the supporting tube 14, and lockable and matching with the connecting base 16; a pressurizing device connector 19 configured for expanding or retracting the expandable sacculus 12 through the connecting base 16; and a brain depressor 20 which is sheathed on a distal end of the flexible retractor component 10 and enters the avulsed passageway opened by the flexible retractor component 10 so as to make the avulsed passageway remain an avulsed status, and thereby avoid the avulsed passageway from being retracted.

In the brain retractor device provided by the embodiment of the present invention, the lock mechanism 18 is pushed into the connecting base 16, and thus the expandable sacculus 12 is stretched and becomes slim, and the length and the position of the expandable sacculus 12 that enter the diseased region can be controlled. Moreover, by connecting a pressurized device (e.g., an injector) to the pressurizing device connector 19 and injecting liquid into the pressurizing device connector 19, the expanded sacculus 12 is expanded, and when liquid is discharged, the expandable sacculus 12 is retracted. By quantitatively controlling the amount of the liquid injected by the pressurized device (e.g., the injector) and the pressurizing device connector 19, the expanded diameter of the expandable sacculus 12 is accurately controlled, and thus the size and the position of the avulsed passageway is accurately controlled, such that the injury to brain tissue during the avulsing process can be reduced, the amount of bleeding in the surgery can be reduced, and cranial nerves can be furthest protected.

Specifically, a volume of the expandable sacculus 12 that has been expanded can be calculated, such that the amount of the liquid that needs to be injected by the pressurized device (e.g., the injector) and the pressurizing device connector 19 during a surgery process can be obtained, thereby facilitating injecting quantitative liquid into the pressurized device (e.g., the injector) beforehand.

The brain retractor device provided by the embodiment of the present invention can substitute a manual operation, establish a standard retractor model, and reduce the difficulty of neurosurgery. Moreover, the brain retractor device reduces the requirements for professional skills and experiences of surgery operators, reduces labour intensity, reduces noneffective exposure, shortens the surgery time, and reduces traumas. The surgery can get rid of the limitation of body positions, and it is convenient for a doctor to operate.

In this embodiment, the material of the expandable sacculus 12 can be natural latex, polyurethane (as abbreviated as PU), thermoplastic elastomer high polymer material, silica gel, or the like. According to requirements, the expandable sacculus 12 can be a compliant sacculus or semi-compliant sacculus.

It needs to explained that, "the proximal end" and "the distal end" described in each embodiment of the present invention are meaningful when the brain retractor device is used by a surgery operator, wherein, "a proximal end" is referred to as an end close to the surgery operator, "a distal end" is referred to as the end far away from the surgery operator, and they are not intended to limit the present invention.

Further, the brain retractor device provided by the embodiment of the present invention can control the expanded diameter of the expandable sacculus 12 so as to accurately control the size of the avulsed passageway, such that the injury to brain tissue in the avulsing process can be reduced. In other embodiments, the expanded diameter of the expandable sacculus 12 can also be arranged to be any size according to actual needs.

Furthermore, the expandable sacculus 12 that has been inflated can be cylindrical, dumbbell-shaped, peanut-shaped, or cone-shaped. The expandable sacculus 12 can have substantially the same size from the proximal end to the distal end, and thus have a cylinder-shape, a drum-shape, or other similar shapes. The size of the proximal end of the expandable sacculus 12 can be slightly larger than the size of the distal end of the expandable sacculus 12, such that the expandable sacculus 12 has a dumbbell-shape or other similar shapes. The shape of the proximal end of the expandable sacculus 12 can be substantially the same as the shape of the distal end of the expandable sacculus 12, and the size of the expandable sacculus 12 can shrink and become smaller and smaller between the proximal end and the distal end, such that the expandable sacculus 12 has a peanut-shape or other similar shapes. The size of the expandable sacculus 12 can gradually reduces from the proximal end to the distal end, such that the expandable sacculus 12 has a cone-shape or other similar shapes. In other embodiments, the shape of the expandable sacculus 12 is not limited thereto, and can be any other structural shapes that can achieve expansion and retraction.

Please refer to FIGS. 1-2, furthermore, a proximal end of the connecting base 16 is provided with a bayonet 160 that matches with the lock mechanism 18, the lock mechanism 18 comprises a push rod 180 that runs through the connecting base 16 and stretches into the supporting tube 14 and the expandable sacculus 12 sequentially, a pushing member 182 connected with the push rod 180, and a fixture block 184 protruded from an outer side of a front end of the pushing member 182 so as to match with the bayonet 160. In the brain retractor device provided by the embodiment of the present invention, the fixture block 184 of the lock mechanism 18 matches with the bayonet 160, such that the push rod 180 is locked and fixed on the connecting base 16; simultaneously, the push rod 180 stretches into the supporting tube 14 and the expandable sacculus 12 along the connecting base 16, such that the expandable sacculus 12 is stretched and becomes slim. By a cooperation between the size of the push rod 180 and the diameter and the length of the expandable sacculus 12, the length and the position of the expandable sacculus 12 that enter the diseased region can be accurately controlled, and the injury to brain tissue can be reduced to the maximum extent.

In this embodiment, the connecting base 16 can be single-chambered, Y type double-chambered, inverted T-typed double-chambered, three-chambered, or four-chambered, and the connecting base 16 can be made of plastic materials, such as PC (Poly Carbonate), ABS (Acrylonitrile Butadiene Styrene) and so on, or of medical macromolecular materials.

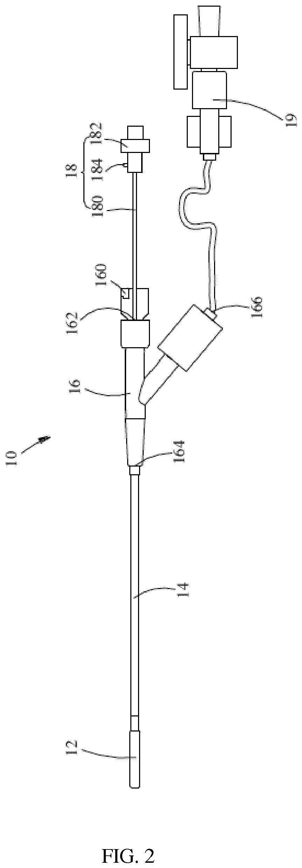

Please refer to FIG. 1 and FIG. 2, in this embodiment, the connecting base 16 is double-chambered and has a lot of chambers. Specifically, the connecting base 16 has a first chamber opening 162 connected with the lock mechanism 18, a second chamber opening 164 that communicates with the first chamber opening 162 and connects with the supporting tube 14, and a third chamber opening 166 connected with the pressurizing device connector 19, the third chamber opening 166 is communicated with the second chamber opening 164; when injecting liquid into the pressurizing device connector 19, the liquid enters the second chamber opening 164 through the third chamber opening 166, further enters the supporting tube 14, and flows into the expandable sacculus 12 so as to make the expandable sacculus 12 be inflated. Inversely, when the liquid is discharged from the pressurizing device connector 19, the expandable sacculus 12 is retracted. The pressurizing device connector 19 is provided with an injection pipe connected with the third chamber opening 166, the expandable sacculus 12 can be inflated when an injector is used to inject liquid into the injection pipe, and the expandable sacculus 12 can be retracted when liquid is discharged from the injection pipe.

In this embodiment, the lock mechanism 18 is a bayonet-typed lock mechanism, the push rod 180, the pushing member 182 and the fixture block 184 are made integrally, and the material of the lock mechanism 18 is selected from stainless steel, plastic material, and medical macromolecular materials.

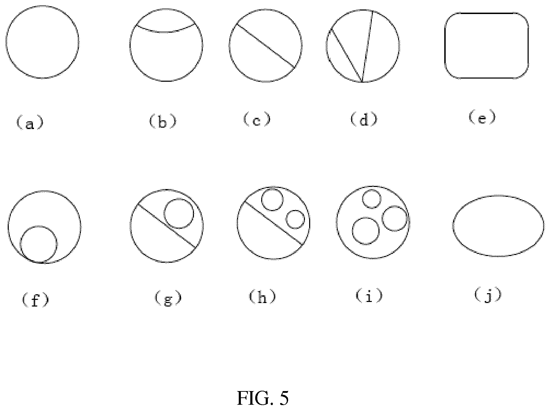

Please refer to FIG. 5, furthermore, a tube body of the supporting tube 14 is cylindrical or cone-shaped. The supporting tube 14 appears as a cylindrical shape or a conical shape from the proximal end thereof to the distal end thereof, and can also be in other shapes.

Furthermore, the distal end of the supporting tube 14 is connected with the proximal end of the expandable sacculus 12, the supporting tube 14 itself has enough intensity and toughness to prevent the supporting tube 14 from being broken. The proximal end of the supporting tube 14 is connected with connecting base 16 and has enough rigidity to bear the push rod 180 of the lock mechanism 18, thereby providing convenience for a user to push and operate the expandable sacculus 12.

In this embodiment, the material of the supporting tube 14 can be PVC (polyvinyl chloride), PU (Poly Urethane), nylon, silica gel, stainless steel, or the like.

In this embodiment, the supporting tube 14 is single-chambered or multi-chambered, as shown in the figures.

In this embodiment, an outer surface of the supporting tube 14 is provided with scale marks, and the outer surface is further provided with coated layer; the coated layer is made of hydrophilic material, teflon, silicon oil, or the like.

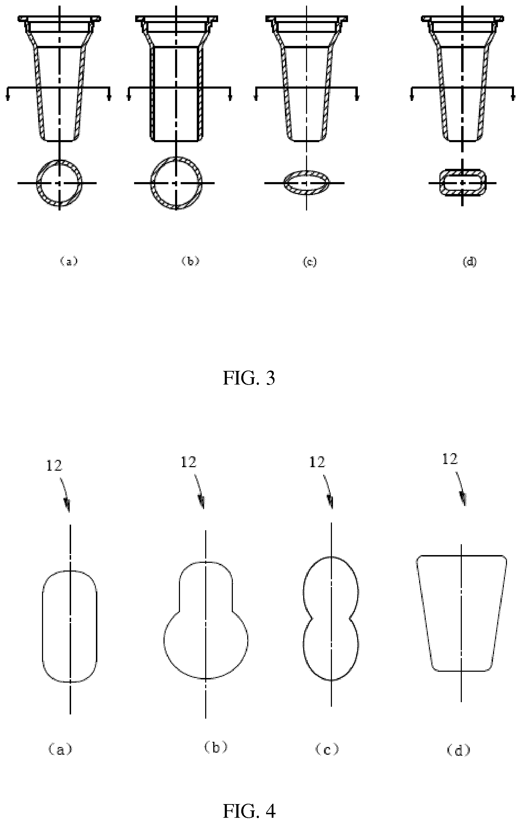

Please refer to FIG.3, furthermore, the shape of the brain depressor 20 can be cylindrical, cone-shaped, elliptic column-shaped, elliptic cone-shaped, square column-shaped with four rounded angles, or square cone-shaped with four rounded angles. The brain depressor 20 is shaped as a cylinder, a cone, an ellipse, or a square cylinder structure with four rounded angles from the proximal end thereof to the distal end thereof.

In this embodiment, the material of the brain depressor 20 can be medical macromolecular material, stainless steel or the like, an outer surface and/or inner surface of the brain depressor 20 is/are provided with (a) coated layer(s), and the material of the coated layer(s) can be hydrophilic material, teflon, silicon oil, or the like. In use, the brain depressor should be aseptically processed.

The brain retractor device provided by the embodiment of the present invention is a one-off tubular retractor for a craniocerebral operation, according to creek characteristics of brain tissue, and by inflating and expanding the expandable sacculus 12, avulsion of the brain tissue can be implemented, and a working passageway for an intracranial surgical operation can be established.

Due to the use of the soft expandable sacculus 12, the brain retractor device provided by the embodiment of the present invention substantially does no damage to the blood vessels and the nerves when the brain depressor 20 is inserted into the brain tissue.

Please refer to FIGS. 1-8, in use, the expandable sacculus 12 of the flexible retractor component 10 is used to perform an accurate control, so that a slow, repeated and accurate expansion of the avulsed passageway is achieved; finally, the brain depressor 20 is sheathed on the distal end of the flexible retractor component 10 and is slowly pushed into the avulsed passageway that has already been expanded. In the process of pushing the brain depressor 20, the expandable sacculus 12 is appropriately inflated and retracted, so that it is easier for the tubular brain depressor 20 to enter the avulsed passageway. The brain retractor device provided by the embodiment of the present invention can reduce the brain retraction injury in the process of avulsing the brain, avoids evoking of angiorrhexis and excessive avulsions and cracks of the brain tissue, and reduces secondary bleeding and edema. After the avulsed passageway is avulsed, the brain depressor 20 is placed in the avulsed passageway, such that the avulsed brain tissue is prevented from being retracted and collapsed, the operable time for the surgery can be extended, and an accidental injury to the brain tissue can be prevented when other medical instruments enters the avulsed passageway.

A specific example of using the brain retractor device provided by the embodiment of the present invention is given below:
The flexible brain retractor component 10 is inserted from a bone window opening, an injector is used to slowly inject liquid into the pressurizing device connector 19, the liquid is repeatedly injected and extracted, and repeated expansions and retractions of the expandable sacculus 12 are achieved. After the expansion of the expandable sacculus 12 is completed, the avulsed passageway is formed. The brain depressor 20 is sheathed on the distal end of the flexible retractor component 10 and is slowly pushed into the expanded avulsed passageway. In the process of pushing the brain depressor 20, the expandable sacculus 12 is appropriately inflated and retracted, so it is easier for the tubular brain depressor 20 to enter the avulsed passageway. When the tubular brain depressor 20 arrives at a predetermined position, the liquid in the expandable sacculus 12 of the flexible retractor component 10 is discharged, and then the flexible retractor component 10 is removed.

Example one: in case of brain bleed, hematoma under the cranial nerves is removed; after the surgery, the patient sobers up immediately.

When the brain retractor device provided by the embodiment of the present invention is used to perform the surgery, a bone window is opened on a skull, a brain needle is inserted from the bone window and reaches a hematoma region, then the brain needle is removed and the flexible retractor component 10 is inserted into the brain tissue; the expandable sacculus 12 is repeatedly and slowly expanded and retracted until the brain tissue is completely avulsed, so that the avulsed passageway is formed. The brain depressor 20 is sheathed on the distal end of the expandable sacculus 12 of the flexible retractor component 10, the expandable sacculus 12 is expanded and retracted and the brain depressor 20 is placed inside the avulsed passageway. Normal saline is used for washing away the hematoma. The brain depressor 20 is removed and the bone window is sutured.

Specifically, the example one can be accomplished by three implementation schemes as follows:

### Scheme one:

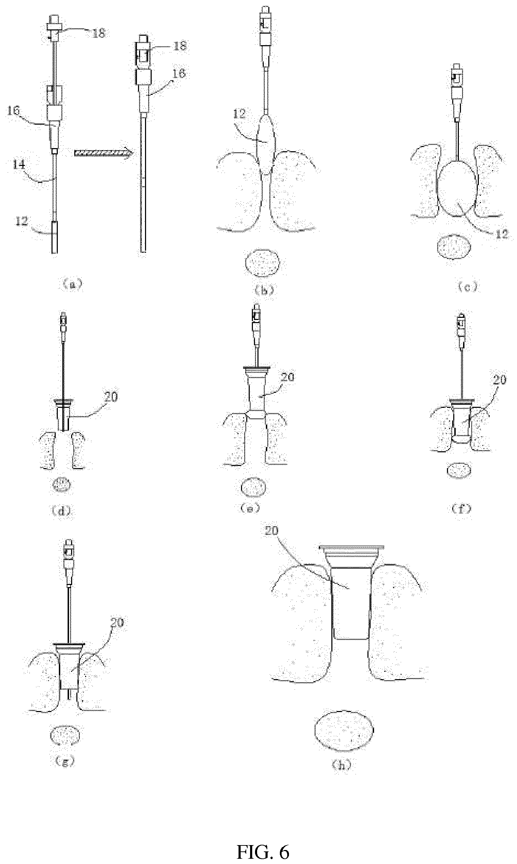

Please refer to FIG. 6, the lock mechanism 18 of the flexible retractor component 10 is locked and connected with the connecting base 16, and the push rod 180 is stretched into the expandable sacculus 12 along the supporting tube 14 so as to make the expandable sacculus 12 of the flexible retractor component 10 be stretched and become slim, so that it is convenient for the flexible retractor component 10 to enter.

The flexible retractor component 10 is inserted into the brain tissue, and an injector is used for injecting normal saline into the flexible retractor component 10 through the pressurizing device connector 19, thus a radial diameter of the expandable sacculus 12 can be accurately controlled, such that the brain tissue is slowly expanded; at the same time, the flexible retractor component 10 gradually enters the diseased region. When the flexible retractor component 10 arrives at the diseased region, it is slowly and repeatedly expanded, such that the brain tissue is avulsed completely, and the avulsed passageway is formed.

In an expanding process, an expanded size of the expandable sacculus 12 can be accurately controlled.

After expansion of the expandable sacculus 12 is finished, the expandable sacculus 12 is retracted so that the flexible retractor component 10 is removed, then the brain depressor 20 is sheathed on the distal end of the expandable sacculus 12 of flexible retractor component 10, the expandable sacculus 12 is expanded and retracted, and the brain depressor 20 is placed inside the flexible retractor component 10.

When the brain depressor 20 arrives at the diseased region, the expandable sacculus 12 is retracted, and the flexible retractor component 10 is removed, at this time, an inner hole of the brain depressor 20 serves as a passageway for surgery.

When the operation is completed, the brain depressor 20 is removed, and the operation is finished.

### Scheme two:

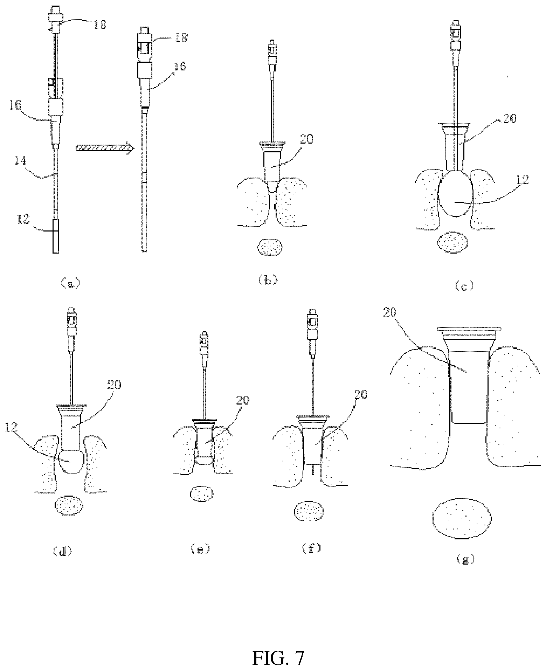

Please refer to FIG. 7, the lock mechanism 18 of the flexible retractor component 10 is locked and connected with the connecting base 16 and the push rod 180 is stretched into the expandable sacculus 12 along the supporting tube 14 to make the expandable sacculus 12 of the flexible retractor component 10 be stretched and slim, so that it is convenient for the flexible retractor component 10 to enter.

The flexible retractor component 10 is inserted into the brain tissue, the brain depressor 20 is sheathed on the distal end of the flexible retractor component 10, and an injector is used to inject normal saline into the flexible retractor component 10 through the pressurizing device connector 19, thus a radial diameter of the expandable sacculus 12 can be accurately controlled, and the brain tissue is slowly expanded; at the same time, the flexible retractor component 10 gradually enters the diseased region. When the flexible retractor component 10 arrives at the diseased region, it is slowly and repeatedly expanded, such that the brain tissue is avulsed completely, and the avulsed passageway is formed.

In the expanding process, the expanded size of the expandable sacculus 12 can be accurately controlled.

After the expansion is finished, the expandable sacculus 12 of the flexible retractor component 10 is slowly retracted, at the same time, the brain retractor device is introduced into the brain tissue slowly along the flexible retractor component 10.

When the brain depressor 20 arrives at the diseased region, the expandable sacculus 12 of the flexible retractor component 10 is completely retracted, and the flexible retractor component 10 is removed, at this time, an inner hole of the brain depressor 20 serves as a passageway for operation.

When the operation is completed, the brain depressor 20 is removed, and the operation is finished.

### Scheme three:

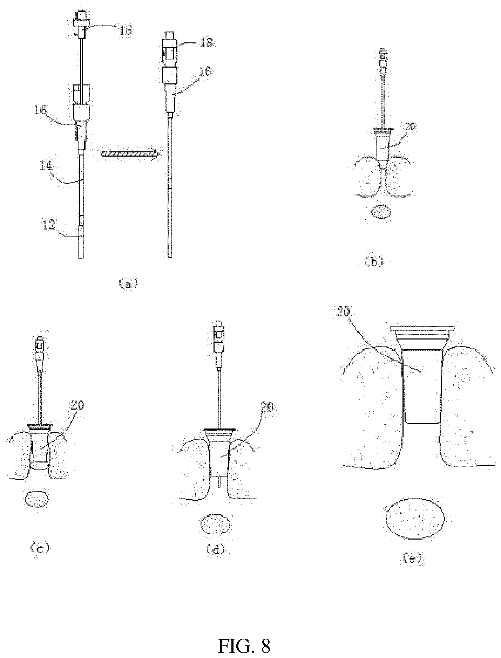

Please refer to FIG. 8, the lock mechanism 18 of the flexible retractor component 10 is locked and connected with the connecting base 16, and the push rod 180 is stretched into the expandable sacculus 12 along the supporting tube 14 to make the expandable sacculus 12 of the flexible retractor component 10 be stretched and become slim, so that it is convenient for the flexible retractor component 10 to enter.

The brain depressor 20 is sheathed on the flexible retractor component 10; while the flexible retractor component 10 is inserted into the brain tissue, an injector is used to slowly inject normal saline into the flexible retractor component 10 through the pressurizing device connector 19, in this way, a radial diameter of the expandable sacculus 12 can be accurately controlled, and the brain tissue can be slowly expanded.

While the flexible retractor component 10 is expanded, the brain depressor 20 is maintained to gradually enter the diseased region.

When the brain depressor 20 arrives at the diseased region, the expandable sacculus 12 of the flexible retractor component 10 is completely retracted, and the flexible retractor component 10 is removed, at this time, an inner hole of the brain depressor 20 serves as the passageway for surgery.

After the operation is completed, the brain depressor 20 is removed, and the operation is finished.

In another preferred embodiment, as shown in FIG. 9, one end of the expandable sacculus 12 is fixedly connected to the push rod 180, the other end of the expandable sacculus 12 is fixedly connected to the supporting tube 14, and the push rod 180 and the supporting tube 14 are arranged coaxially. When the push rod 180 is pushed, since the supporting tube 14 is immovable, the other end of the expandable sacculus 12 fixed on the supporting tube 14 is immovable too, and the push rod 180 drives the end of the expandable sacculus 12 fixed on the push rod 180 to move. According to this design, the push rod 180 can be pushed before operation, so that the expandable sacculus 12 is driven to stretch (i.e., expand in the length direction thereof) to a designated position, while the end of the expandable sacculus 12 fixed with the supporting tube 14 is immovable, such that the expandable sacculus 12 has no axial expansion during the surgery, and the brain tissue is prevented from an accidental injury. If both two ends of the expandable sacculus 12 are fixed on a same object (e.g., fixed on the push rod 180), with the expansion of the expandable sacculus 12, the length of the expandable sacculus 12 may be slightly changed, and the diameter of the expandable sacculus 12 may also be changed simultaneously, such that the shape of the expanded expandable sacculus 12 is uncontrollable. If the two ends of the expandable sacculus 12 are fixed on different objects respectively (e.g., fixed respectively on the push rod 180 and the supporting tube 14 that are coaxial), but the lock mechanism 18 comprising the bayonet 160 and the fixture block 184 is absent, before use, it is difficult to expand the expandable sacculus 12 to a certain length beforehand and keep the expanded shape of the expandable sacculus 12; thus, with the expansion of the expandable sacculus 12, the length of the expandable sacculus 12 can be slightly changed, the diameter of the expandable sacculus 12 may also be changed simultaneously, and the shape of the expanded expandable sacculus 12 is also uncontrollable.

By means of a particular component, the expandable sacculus 12 can be fixed on the push rod 180 and the supporting tube 14. For example, as shown in FIGS. 9-11, the distal end of the expandable sacculus 12 near the flexible retractor component 10 is fixed on the push rod 180 through a first fastening piece 222, the proximal end of the expandable sacculus 12 near the flexible retractor component 10 is fixed on the supporting tube 14 through a second fastening piece 224, and the first fastening piece 222 and the second fastening piece 224 are sheathed on the distal end and the proximal end of the expandable sacculus 12 respectively. By means of the two fastening pieces, the expandable sacculus 12 is fixed on the push rod 180 and the supporting tube 14, and thus the expandable sacculus 12 can be fixed more stably. When the push rod 180 is pushed towards the distal end of the expandable sacculus 12, since the supporting tube 14 is immovable, the other end of the expandable sacculus 12 fixed on the supporting tube 14 is immovable too, the push rod 180 is pushed towards the distal end and drives the end of the expandable sacculus 12 fixed on the push rod 180 to move. According to this design, before operation, the push rod 180 can be pushed towards the distal end, and the expandable sacculus 12 is driven to stretch (i.e., expand in the length direction thereof) to the designated position, the end of the expandable sacculus 12 fixed on the supporting tube 14 is immovable, such that the expandable sacculus 12 has no axial expansion during the surgery, and the brain tissue can be avoided from accidental injury.

Both the first fastening piece 222 and the second fastening piece 224 can be hollow and cylindrical, and the hollow part of the first fastening piece 222 and the second fastening piece 224 are sheathed on the distal end (and the push rod 180) and the proximal end (and the supporting tube 14) of the expandable sacculus 12, the cylinder design is more suitable for cylindrical structures of the push rod 180 and the supporting tube 14. The material of the first fastening piece 222 and the second fastening piece 224 can be stainless steel or similar material that possesses certain rigidity, which is not prone to be deformed and has a preferable immobilization performance.

A part of the distal end of the expandable sacculus 12 near the flexible retractor component 10 is bent towards the inside of the expandable sacculus 12, and the first fastening piece 22 is sheathed on the bent part of the expandable sacculus 12 that is inside the expandable sacculus 12; the second fastening piece 224 is sheathed on an outer side of a part of the proximal end of the expandable sacculus 12 near the flexible retractor component 10. As for the part of the distal end of the expandable sacculus 12 near the flexible retractor component 10, by the method of bending the part of expandable sacculus 12 towards the inside of the expandable sacculus 12, and then sheathing the first fastening piece 222 on the bent part of the expandable sacculus 12 that is inside the expandable sacculus 12 so as to stabilize the expandable sacculus 12, the first fastening piece 222 can be unexposed, and the brain tissue can be avoided from accidental injury during the surgery.

When the brain retractor device is used, the pushing member 182 is pushed and the push rod 180 is driven to move towards the distal end of the flexible retractor component 10, the expandable sacculus 12 is expanded in the length direction. When the pushing member 182 is pushed and the push rod 180 is driven to move towards the distal end of the flexible retractor component 10 until the fixture block 184 is embedded in the bayonet 160, and the expandable sacculus 12 is expanded by the pressurizing device, the expandable sacculus 12 is merely expanded radially.

The inner part of the push rod 180 near the distal end of the flexible retractor component 10 is filled with inner core 186, so that the brain tissue can be prevented from entering inner hole of the push rod 180 during the surgery. Preferably, the inner core material is stainless steel.

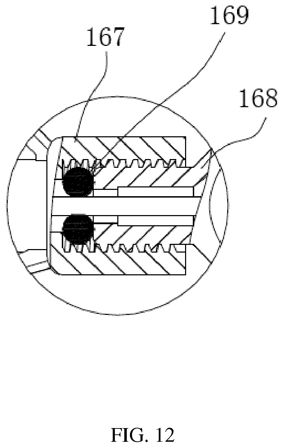

As shown in FIG. 9 and FIG. 12, the connecting base 16 comprises a sealed base body 167 and a nut cap 168, both an interior of the sealed base body 167 and an exterior of the nut cap 168 are provided with threads, and the thread of the sealed base body 167 and the thread of the nut cap 168 match with each other; the nut cap 168 is threadedly connected in the sealed base body 167, and the push rod 180 stretches into the nut cap 168 and the sealed base body 167 sequentially. A room formed by a part of the nut cap 168 near the distal end of the flexible retractor component 10, the interior of the sealed base body 167, and the exterior of the push rod 180 is provided therein with a seal ring 169. With the aforementioned sliding sealing structure, since the interior of the push rod 180 is hollow, an air chamber is formed in the push rod 180, and the air chamber can achieve an exhausting function during the surgery so as to balance the air pressure.

The expandable sacculus 12 in this embodiment is implemented by the sliding lock mechanism, in storing and transportation processes, the expandable sacculus 12 lies in a natural state (i.e., an unexpanded state), so that the expandable sacculus 12 is well protected. In use, the pushing member 182 is pushed firstly, the expandable sacculus 12 is stretched and the length of the expandable sacculus 12 is maintained by the lock mechanism 18 comprising the bayonet 160 and the fixture block 184, so that the expandable sacculus 12 can only be expanded in the diameter direction subsequently. During a surgery, an expansion position of the expandable sacculus 12 can be accurately located and the expanded shape of the expandable sacculus 12 can be controlled, such that the expandable sacculus 12 has no axial expansion and is only expanded radially when it is inflated, and thus the brain tissue can be avoided from accidental injuries.

Dimensionally, by calculating a relationship between the original length of the expandable sacculus 12 and the length of the expandable sacculus 12 that has been expanded, the expanded length of the expandable sacculus 12 can be determined, and it is ensured that the expandable sacculus 12 has been correctly stretched before it is expanded. For example, a length of the expandable sacculus 12 in an original state is from 12mm to 16 mm (e.g., 12 mm, 13mm, 15mm, 16mm), and a length of the expandable sacculus 12 that has been stretched and stabilized is from 48 mm to 52 mm (e.g., 48mm, 50mm, 52mm).

Another embodiment of the present invention further provides a surgical retractor device comprising:
a flexible retractor component which comprises an expandable sacculus that is made of elastic material and can be retracted so as to enter a diseased region and form an avulsed passageway for the diseased region; a supporting tube communicated with the expandable sacculus; a connecting base, wherein the connecting base and the expandable sacculus are located at two ends of the supporting tube respectively; a lock mechanism arranged coaxially with the supporting tube, and lockable and matching with the connecting base, and a pressurizing device connector configured for expanding or retracting the expandable sacculus through the connecting base.

The specific implementation and the principle of this embodiment are similar to that of the aforementioned embodiment, and are not repeatedly described herein.

In the surgical retractor device provided by the embodiment of the present invention, the lock mechanism 18 is pushed into the connecting base 16 to make the expandable sacculus 12 be stretched and becomes slim, such that the length and the position of the expandable sacculus 12 that enter the diseased region are controlled. Moreover, by connecting a pressurizing device (e.g., an injector) to the pressurizing device connector and injecting liquid into the pressurizing device connector, the expandable sacculus 12 is expanded; and when liquid is discharged, the expandable sacculus is retracted. By quantitatively controlling the amount of liquid injected by the pressurizing device (e.g., the injector) into the pressurizing device connector, the expanded diameter of the expandable sacculus 12 is accurately controlled, and thus the size and the position of the avulsed passageway is accurately controlled, such that injury to brain tissue during the avulsing process can be reduced, the amount of bleeding in the surgery can be reduced, and cranial nerves can be protected to the maximum extent.

The aforementioned embodiments are only preferred embodiments of the present application and should not be intended to limit the present invention. Any modification, equivalent replacement, improvement, and so on, which are made within the spirit and the principle of the present invention, should be included in the protection scope of the present invention.

## Claims

1. A brain retractor device comprising:
a flexible retractor component (10) which comprises an expandable sacculus (12) that is made of elastic material and can be retracted so as to enter a diseased region and form an avulsed passageway for the diseased region; a supporting tube (14) communicated with the expandable sacculus (12); a connecting base (16), wherein the connecting base (16) and the expandable sacculus (12) are located at two ends of the supporting tube (14) respectively; a lock mechanism (18) arranged coaxially with the supporting tube (14) and lockable and matching with the connecting base (16); a pressurizing device connector (19) configured for expanding or retracting the expandable sacculus (12) through the connecting base (16); and
a brain depressor (20) which is sheathed on a distal end of the flexible retractor component (10) and enters the avulsed passageway opened by the flexible retractor component (10) so as to make the avulsed passageway remain at an avulsed status.

2. A surgical retractor device comprising:
a flexible retractor component (10) which comprises an expandable sacculus (12) that is made of elastic material and can be retracted so as to enter a diseased region and form an avulsed passageway for the diseased region; a supporting tube (14) communicated with the expandable sacculus (12); a connecting base (16), wherein the connecting base (16) and the expandable sacculus (12) are arranged at two ends of the supporting tube (14) respectively; a lock mechanism (18) arranged coaxially with the supporting tube (14), lockable and matching with the connecting base (16); and a pressurizing device connector (19) configured for expanding or retracting the expandable sacculus (12) through the connecting base (16).

3. The device according to claim 1 or claim 2, wherein the expandable sacculus (12) is a compliant sacculus or non-compliant sacculus.

4. The device according to claim 1 or claim 2, wherein the expandable sacculus (12) is cylindrical, dumbbell-shaped, peanut-shaped, or cone-shaped.

5. The device according to any one of claims 1 to 4, wherein a proximal end of the connecting base (16) is provided with a bayonet (160) that matches with the lock mechanism (18), the lock mechanism (18) comprises a push rod (180) that runs through the connecting base (16) and sequentially stretches into the supporting tube (14) and the expandable sacculus (12), a pushing member (182) connected with the push rod (180), and a fixture block (184) protruded from an outer side of a front end of the pushing member (182) so as to match with the bayonet (160).

6. The device according to claim 5, wherein the supporting tube (14) and the push rod (180) are arranged coaxially.

7. The device according to claim 5 or claim 6, wherein one end of the expandable sacculus (12) is fixed to the push rod (180), and the other end of the expandable sacculus (12) is fixed to the supporting tube (14).

8. The device according to claim 7, wherein a distal end of the expandable sacculus (12) near the flexible retractor component (10) is fixed to the push rod (180) through a first fastening piece (222), a proximal end of the expandable sacculus (12) near the flexible retractor component (10) is fixed to the supporting tube (14) through a second fastening piece (224), and the first fastening piece (222) and the second fastening piece (224) are respectively sheathed on the distal end and the proximal end of the expandable sacculus (12).

9. The device according to claim 8, wherein a part of the distal end of the expandable sacculus (12) near the flexible retractor component (10) is bent towards the inside of the expandable sacculus (12), and the first fastening piece (222) is sheathed on the bent part of the expandable sacculus (12) inside the expandable sacculus (12); the second fastening piece (224) is sheathed on an outer side of the part of the proximal end of the expandable sacculus (12) near the flexible retractor component (10).

10. The device according to claim 7 or claim 8, wherein when the pushing member (182) is pushed, and drives the push rod (180) to move towards the distal end of the flexible retractor component (10), the expandable sacculus (12) is expanded in a length direction; when the pushing member (182) is pushed and drives the push rod (180) to move towards the distal end of the flexible retractor component (10) until the fixture block (184) is embedded in the bayonet (160), and when the expandable sacculus (12) is expanded by the pressurizing device (19), the expandable sacculus (12) is only expanded radically.

11. The device according to claim 7 or claim 8, wherein an inner part of the push rod (180) near a distal end of the flexible retractor component (10) is filled with inner core (186).

12. The device according to any one of claims 5 to 12, wherein the connecting base (16) comprises a sealed base body (167) and a nut cap (168), both an interior of the sealed base body (167) and an exterior of the nut cap (168) are provided with threads, and the thread of the sealed base body (167) and the thread of the nut cap (168) match with each other; the nut cap (168) is threadedly connected inside the sealed base body (167), the push rod (180) stretches into the cap (168) and the sealed base body (167) sequentially, and a room formed by a part of the cap (168) near the distal end of the flexible retractor component (10), the interior of the sealed base body (167), and an exterior of the push rod (180) is provided therein with a sealing ring (169).
